Europäisches Patentamt

European Patent Office

Office européen des brevets

(11) Publication number: **0 228 243 B1**

(12) EUROPEAN PATENT SPECIFICATION

(45) Date of publication of patent specification: **25.03.92**  (51) Int. Cl.⁵: **G01N  33/577**, G01N 33/574,
A61K 39/395, A61K 49/02

(21) Application number: **86309857.0**

(22) Date of filing: **17.12.86**

㊴ Monoclonal antibodies having binding specificity to human prostate tumor-associated antigens
and methods for employing the same.

(30) Priority: **17.12.85 US 809719**
         **15.12.86 US 941911**

(43) Date of publication of application:
   **08.07.87 Bulletin  87/28**

(45) Publication of the grant of the patent:
   **25.03.92 Bulletin  92/13**

(84) Designated Contracting States:
   **AT BE CH DE ES FR GB GR IT LI LU NL SE**

(56) References cited:

   **CANCER RESEARCH, vol. 42, no. 4, April
   1982; J.L.WARE et al., pp. 1215-1222**

   **HYBRIDOMA, vol. 4, no. 1, 1985; J.LINDGREN
   et al., pp. 37-45**

(73) Proprietor: **Eastern Virginia Medical Authority
   P.O. Box 1980
   Norfolk Virginia 23501(US)**

(72) Inventor: **Wright, George L., Jr.
   829 Moultrie Ct.
   Virginia Beach, VA 23455(US)**
   Inventor: **Starling, James J.
   1126 Hillcrest Drive
   Carmel, IN 43032(US)**

(74) Representative: **Woodcraft, David Charles
   BROOKES & MARTIN High Holborn House
   52/54 High Holborn
   London, WC1V 6SE(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### FIELD OF THE INVENTION

The present invention relates to monoclonal antibodies having binding specificity to human prostate tumor-associated antigens but not to prostate-specific antigen (PSA) or prostatic acid phosphatase (PAP); and methods of diagnosis and treatment employing the same.

### BACKGROUND OF THE INVENTION

Various monoclonal antibodies have been developed over the years in an attempt to find substances which have binding specificity to tumor-associated antigens. It has been hoped that these monoclonal antibodies would be useful in the diagnosis and treatment of tumors.

Monoclonal antibodies are produced by cell lines known as hybridomas. Hybridomas can be created using tumor tissue such that the monoclonal antibodies produced therefrom have binding affinity to antigens associated with the tumor tissue. Most monoclonal antibodies are disadvantageous in that they have either (1) an extreme binding specificity, i.e., they are not capable of binding to many tumor types other than those which are very closely related to the tumor from which they were developed or (2) an extreme lack of binding specificity, i.e. they bind to almost all types of cells whether malignant or non-malignant. As a result, these monoclonal antibodies are generally not clinically useful because due to (1) their extreme binding specificity, a unique monoclonal antibody needs to be developed for each tumor to be diagnosed or treated, or (2) their extreme lack of binding specificity, the monoclonal antibody is incapable of differentiating between malignant and non-malignant cells.

In spite of the concerns which have arisen regarding the usefulness of monoclonal antibodies in diagnostic and therapeutic methods, it is still believed that monoclonal antibodies can be developed which are organ specific, i.e., capable of binding to antigens associated with only a particular organ, but which, given their organ-specificity, are capable of binding to an antigen that is associated with many different types of tumors arising in the particular organ.

Prostate carcinoma is one of the most prevalent malignancies among men in the United States. It is postulated that the disease is often present but undetected in the male population. Moreover, it has been noted that prostate carcinoma often exhibits a long latency period. Further, prostate carcinoma is known for its high metastatic potential. For these reasons, it has been desired to develop a substance or substances useful for diagnosing, in vivo or in vitro, the presence of prostate tumors and for treating such tumors, especially once the tumors have metastasized.

Various monoclonal antibodies to prostate antigenic markers have been previously described. These previously-described monoclonal antibodies bind to either prostate-specific antigen (PSA) (Wang, M.C. et al, Urol., 17:159-169 (1979)), or prostatic acid phosphatase (PAP) (Chu, T.M. et al, Urol., 15:319-322 (1978)), or both. These previously-described monoclonal antibodies are disadvantageous because they fail to bind to some prostate tumors.

### SUMMARY OF THE INVENTION

Accordingly, it is an object of the present invention to provide a monoclonal antibody having binding specificity to human prostate tumor-associated antigens but which do not bind to PSA or PAP.

Another object of the present invention is to provide a monoclonal antibody which bind to antigens associated with all prostate tumors and has little or no binding to cells originating from organs other than the prostate.

An additional object of the present invention is the use of the said antibody in the manufacture of medicaments, and in a method for diagnosing prostate tumors and metastases thereof comprising exposing a sample of body fluid to the said monoclonal antibody.

Additional objects and advantages of the present invention will be apparent from the detailed description of the invention provided hereinafter.

### BRIEF DESCRIPTION OF THE DRAWINGS

Figures 1A and 1B graphically illustrate the quantitative absorption of monoclonal antibodies TURP-27 and TURP-73, respectively, with crude membrane preparations of human tissues.

Figures 2A and 2B graphically illustrate the binding inhibition of $^{125}$I-labelled TURP-27 and $^{125}$I-labelled

TURP-73, respectively, by unlabelled monoclonal antibodies directed against prostate antigens.

Figures 3A and 3B graphically illustrate cross-blocking of binding between TURP-27 and TURP-73, respectively, to a Ca 1126 prostate carcinoma crude membrane extract antigen preparation.

## DETAILED DESCRIPTION OF THE INVENTION

In one embodiment, the above-described objects have been met by monoclonal antibodies having binding specificity to human prostate tumor-associated antigens but do not have binding specificity to prostate-specific antigen or prostatic acid phosphatase and having the designation: TURP-27. TURP-27 has been deposited at the American Type Culture Collection, Rockville, Maryland, under Accession No 40292.

Monoclonal antibody TURP-27 is directed at a unique epitope present on malignant prostate tissue. In addition, it is to a clinically acceptable degree, organ-specific or organ-associated for prostate tissues. Thus, the new monoclonal antibody is useful for diagnosing the presence of prostatic tumors and metastases thereof and for treating the same.

According to the invention there is provided a monoclonal antibody having binding specificty to human prostate tumor associated antigens but not having binding specificty to prostate-specific antigen or prostatic acid phosphatase characterised in that the antibody is TURP-27 having the identifying characteristics of ATCC No 40 292.

According to the invention there is provided the aforementioned monoclonal antibody for use as a medicament.

In accordance with the invention the monoclonal antibody optionally conjugated to a marker can be used for the diagnosis of prostate tumors or metastases thereof.

According to the invention there is provided a method for diagnosing prostate tumors and metastases thereof comprising the steps of

a) exposing a sample of body fluid to monoclonal antibody TURP-27,

b) determining the amount of monoclonal antibody binding to substance present in the body fluid; and

c) comparing the amount of monoclonal antibody bound to body fluid substances to a predetermined base level to ascertain the presence of prostate tumors or metastases thereof.

The invention further provides the use of TURP-27 therapeutic agent in the manufacture of a medicament for treating prostate tumors or metastases thereof Monoclonal antibodies having binding specificity to human prostate tumor-associated antigens but not having binding specificity to PSA or PAP can be produced and isolated as follows.

Mice are immunized with prostate tumor preparations. The spleen cells from immunized mice are isolated and fused with mouse myeloma cells and cultured under conditions which allow only for growth of hybridomas. The hybridomas are then screened for production of the desired monoclonal antibody.

Immunization of mice, isolation of the immunized cells, fusion of the isolated cells with mouse myeloma cells and culturing under conditions which allow for growth of hybridomas are all conducted by methods well known or readily determined by those skilled in the art.

For immunization of the mouse spleen cells, a preparation from any tumor having prostate tumor-associated antigens can be used. Preferred tumor preparations include those from benign prostatic hyperplasias and prostate carcinomas, particularly prostate adenocarcinomas.

Further, immunization can be carried out with a preparation from just one tumor or from a mixture of one or more tumors comprising one or more tumor types. In this respect, an especially preferred preparation consists of a mixture of three benign prostatic hyperplasias and one prostate adenocarcinoma.

The mouse myeloma cell line can be any of a number of readily available to those skilled in the art. An especially preferred well known and readily available mouse myeloma cell line is P3-NS-1-Ag4-1 (obtained from NIGMS Human Genetic Mutant Cell Repository, repository number GM3573) (NS-1)).

Screening for production of the desired monoclonal antibody is accomplished by determining restricted binding activity to prostate antigens, i.e., binding to prostatic tumor-associated antigens but not to PSA or PAP, and by the lack of cross-reactivity to normal antigens, including those from normal bladder, breast, colon, kidney, lung, pancreas and liver. To be clinically acceptable, the monoclonal antibody must lack reactivity to PSA and PAP and desirably entirely lack cross-reactivity to non-prostate normal antigens. However, the monoclonal antibody can have some reactivity to non-prostate normal antigens if the antigen is from tissue unrelated to the use of the monoclonal antibody in the clinical application. Assays for restricted binding activity to prostate antigens and cross-reactivity to non-prostate normal antigens are readily determined by those skilled in the art.

The monoclonal antibodies are produced in large quantities by injecting hybridoma cells producing the monoclonal antibodies into the peritoneal cavity of mice, and after an appropriate time, harvesting ascites

3

fluid from the mice which yields a very high titer of homogenous antibody and isolating the monoclonal antibodies therefrom. Alternatively, the antibodies can be produced by culturing monoclonal antibody producing cells in vitro and isolating secreted monoclonal antibodies from the cell culture medium.

Hybridomas expressing monoclonal antibodies designated TURP-27 and TUPR-73 were produced according to the above method. The binding specificity of these two monoclonal antibodies and a comparison of such with known prostate monoclonal antibodies are discussed in more detail in Example 1 below. References to TURP 73 in the accompanying are by way of comparative example and are outside the scope of the present application.

Another embodiment of the present invention involves an in vitro method for diagnosing prostate tumors or matastases.

As described above, a body fluid is obtained from a patient and exposed to the monoclonal antibody of the present invention. The diagnosis is then made by determining the amount of monoclonal antibody binding to substances present in the body fluid and comparing the amount of monoclonal antibody bound to the body fluid substances to a predetermined base level. An amount of bound monoclonal antibody exceeding the base level indicates the likely presence of a prostate tumor or metastases thereof.

The body fluid which can be used in the in vitro method is any body fluid suspected of containing human prostate tumor-associated antigens. Examples thereof include blood, serum, seminal plasma, semen, urine and prostatic fluid. The body fluids can be obtained by methods readily known to or determined by those skilled in the art.

The body fluid is exposed to the monoclonal antibody and the amount of monoclonal antibody bound to substances in the body fluid is determined by means of immunochemical assays well known to those skilled in the art, as described, for example, in Klug, T.L. et al, Cancer Res., 44:1048 (1984), Herlyn, M. et al, J. Clin. Immunol., 2:135 (1982), Metzgar, R.S. et al, Proc. Natl. Acad. Sci., USA, 81:5242 (1984), Papsidero, L.D. et al. Cancer Res., 44:4653 (1984), Hayes, D.F. et al, J. Clin. Invest., 75:1671 (1985), Killian, C.S. et al, J. Natl. Cancer Inst., 76:179 (1986), Killian, C.S. et al, Cancer Res., 45:886 (1985), Hedin, A. et al, Proc. Natl. Acad. Sci., USA, 80:3470 (1983), Pekary, A.E. et al, Clin. Chem., 30:1213-1215 (1984), Bast, R.C. et al, New England J. Med., 309:883-887 (1983) and Bellet, D.H. et al, Proc. Natl. Acad. Sci., USA, 81:3869-3873 (1984),

Examples of suitable well known immunochemical assays include "sandwich" or "two-site" immunoradiometric assays, competitive binding assays such as radioimmunoassays, and enzyme immunoassays, which can use enzyme agents capable of being detected spectrophometrically or spectrofluorometrically.

As an example of one type of immunochemical assay useful in the present invention, is a sandwich immunoradiometric assay (IRMA) in which antigen is measured directly by reacting it with an excess of labelled antibody. In such assays, before the antigen is reacted with labelled antibody, it is insolubilized on an immunoadsorbent which specifically binds the antigen. The immunoadsorbent is formed by affixing a monoclonal antibody of the present invention. In sandwich assays for an antigen which is monomeric, two antibodies which recognize distinct epitopes on the antigen are required so that there is no competition for binding to antigen. One is used to form the immunoadsorbent; the other is used as the labelled tracer. In assays for dimeric or polymeric antigens, the same antibody can be used to form the immunoadsorbent as the labelled tracer.

Sandwich IRMA's may be performed in forward, reverse or simultaneous mode.

In a forward sandwich assay for human prostate tumor-associated antigens, a monoclonal antibody of the present invention is affixed to a solid phase to form an immunoadsorbent specific for human prostrate tumor-associated antigens. A liquid sample containing human prostate tumor-associated antigens is incubated with the immunoadsorbent. Incubation is maintained for a sufficient period of time to allow the human prostate tumor-associated antigens in the liquid sample to bind the immobilized monoclonal antibody on the immunoadsorbent. After this first incubation, the solid phase immunoadsorbent is separated from the incubation mixture. The immunoadsorbent may be washed to remove unbound interferring substances, such as non-specific binding proteins, which may also be present in the liquid sample. The immunoadsorbent containing human prostate tumor-associated antigens bound to immobilized monoclonal antibody is subsequently incubated with labelled monoclonal antibody of the present invention. Again, the incubation is carried out for a period of time and under conditions sufficient to ensure binding of the labelled antibody to the human prostate tumor-associated antigen. After the second incubation, another wash may be performed to remove unbound labelled antibody from the solid phase immunoadsorbent. The labelled antibody bound to the solid phase immunoadsorbent is then measured, and the amount of labelled antibody detected serves as a direct measure of the amount of human prostate tumor-associated antigen.

The sandwich IRMA may also be performed in reverse and simultaneous modes. In reverse mode, an incubation mixture is formed of the liquid sample to be tested and a soluble labelled antibody directed against human prostate tumor-associated antigens. The mixture is incubated, then contacted with a solid phase immunoadsorbent also containing a monoclonal antibody directed against human prostate tumor-associated antigens. After another incubation, the immunoadsorbent is separated from the mixture and the label bound to the immunoadsorbent is taken as an indication of the amount of human prostate tumor-associated antigen in the liquid sample.

In the simultaneous mode, an incubation mixture is formed of the liquid sample, the labelled monoclonal antibody and the solid phase immunoadsorbent. After appropriate incubation, the solid phase immunoadsorbent is separated from the mixture and the label associated with the immunoadsorbent is measured to give an indication of the amount of human prostate tumor-associated antigens in the liquid sample.

For each incubation step in the various formats of the assays, the time and conditions of incubation are selected to ensure maximal binding of human prostate tumor-associated antigens to the immobilized antibody and to labelled antibody.

In addition to the IRMA's described herein, other immunoassays useful in this invention include competitive binding assays such as radioimmunoassays (RIA). One suitable type of RIA is a solid phase RIA.

A solid phase immunoadsorbent is prepared as described for the IRMA.

The immunoadsorbent is then incubated with the liquid sample and a known amount of labelled human prostate tumor-associated antigen for a period of time and under conditions which permit binding of the human prostate tumor-associated antigen to the immunoadsorbent. The immunoadsorbent is separated from the liquid sample and the amount of label associated therewith is assessed. By reference to a preestablished inhibition curve defining the relationship between labelled human prostate tumor-associated antigen associated with the immunoadsorbent, the amount of unlabelled human prostate tumor-associated antigen in the liquid sample is determined.

In the various solid phase assays, the immunoadsorbent is separated from incubation mixtures containing the liquid sample, the labelled antibody or both. Separation can be accomplished by any conventional separation technique such as sedimentation or centrifugation. Preferably, though not necessarily, the immunoadsorbent is washed prior to contacting it, when required, with a second incubation medium and prior to measuring the amount of label associated with the immunoadsorbent. The washing removes non-specific interferring substances or excess labelled antibody which may affect the accuracy and sensitivity of the assay.

At the last step in the in vitro diagnostic method according to the present invention, the amount of monoclonal antibody binding to substances present in the body fluid is compared to a predetermined base level.

The determination of the base level of monoclonal antibody assay binding to be expected is a determination routinely made by those of ordinary skill in the art when defining the parameters necessary for reading of a diagnostic test of this sort. These determinations may be made without undue experimentation, particularly in light of the teachings set forth herein.

Generally, the "base level" is defined as (1) two standard deviations above the means of the normal population, or (2) the level below which 99% of the normal population falls.

TURP-27 can be used in the in vivo diagnosis of of prostrate tumors and metastases thereof.

The method involves administering to a patient a monoclonal antibody of the present invention conjugated to a marker and then detecting the presence of the marker in the patient by exposing the patient to an appropriate detecting device.

Administration and detection of the monoclonal antibody conjugated to a marker as well as methods of conjugation of the antibody to the marker are accomplished by methods readily known to or readily determined by those skilled in the art. See for example, Goldenberg, D.M. et al, New England J. Med., 298:1384-1380 (1978), Goldenberg, D.M. et al, J. A. M. A., 250:630-635 (1903), Goldenberg, D.M. et al, Gastroenterol., 84:524-532 (1983), Siccardi, A.G. et al, Cancer Res., 46:4817-4822 (1986), Epenetos, A.A. et al, Cancer, 55:984-987 (1985), Philben, V.J. et al, Cancer, 57:571-576 (1986), Chiou, R. et al, Cancer Res., 45:6140-6146 (1985) and Hwang, K.M. et al, J. Natl. Cancer Inst., 76:849-855 (1986).

The dosage will vary depending upon the age and weight of the patient, but generally a one time dosage of about 0.1 to 20 mg (of the monoclonal antibody conjugated to a marker) per patient is sufficient. A more preferred dosage is about 1.0 to 2.0 mg (of the monoclonal antibody conjugated to a marker) per patient.

Examples of markers which can be conjugated to the antibody are well known to those skilled in the art and include substances which can be detected by nuclear magnetic resonance imaging, i.e., nuclear magnetic spin-resonance isotopes, and radioactive substances.

A preferred example of a nuclear magnetic spin-resonance isotope is gadolinium (Gd).

Suitable examples of radioactive markers include $I^{125}$, $I^{131}$, $I^{123}$, $In^{111}$, $In^{113}$, $Ga^{67}$, $Ga^{68}$, $Ru^{97}$, $Ru^{103}$, $Hg^{197}$, $Hg^{203}$, and $Tc^{99m}$. $In^{111}$ and $Tc^{99m}$ are preferred due to their low energy and suitability for long range detection.

Detection of radioactive markers is by means of a gamma scintillation camera or the like as described in the references cited above.

Nuclear magnetic imaging devices can be used to detect nuclear magnetic spin-resonance isotope markers.

The invention also provides for the use of the antibodies as a therapeutic agent in the manufacture of a medicament for treatment of a patient afflicted with a prostate tumor or metastases thereof.

Methods of preparing and administering the monoclonal antibody-therapeutic agent as well as suitable dosages are well known to or readily determined by those skilled in the art. Representative protocols are described in the references cited below.

Examples of the monoclonal antibody-therapeutic agents used in therapy include antibodies coupled to drugs such as methotrexate and adriamycin as described in, for example, Deguchi, T. et al, Cancer Res., 46:3751 (1986), Deguchi, T. et al, Fed. Proc., 44:1684 (1985), Embleton, M.J. et al, Br. J. Cancer, 49:559 (1984) and Pimm, M.V. et al, Cancer Immunol. Immunother., 12:125-134 (1982), antibodies coupled to toxins, as described in, for example, Uhr, J.W. et al, Monoclonal Antibodies and Cancer, Academic Press, Inc., pp. 85-98 (1983), Vitetta, E.S. et al, Biotechnology and Biol. Frontiers, Ed. P.H. Abelson, pp. 73-85 (1984) and Vitetta, E.S. et al, Sci., 219:644-650 (1983), antibodies coupled to isotopes, such as $I^{131}$, $Yt^{90}$, $Sc^{47}$, $Cu^{67}$, $Bi^{212}$, $Pb^{212}$ and $At^{211}$, as described in, for example, Goldenberg, D.M. et al, Cancer Res., 41:4353 (1981), Carrasquillo, J.A. et al, Cancer Treat. Rep., 68:317 (1984), Zalcberg, J.R. et al, J. Natl. Cancer Inst., 72:697 (1984), Jones, D.H. et al, Int.J. Cancer, 35:715 (1985), Lange, P.H. et al, Surgery, 98:143 (1985), Kaltovich, F.A. et al, J. Nucl. Med., 27:897 (1986), Order, S.E. et al, Int. J. Radiother. Oncol. Biol. Phys., 8:121 (1982), Courtenay-Luck, N. et al, Lancet, 1:1441 (1984) and Ettinger, D.S. et al, Cancer Treat. Rep., 66:289-297 (1982), heterobifunctional antibodies, for example, antibodies coupled or spliced to another antibody so that the complex binds both to the tumor and the body's own killer cells such as T cells, as described, for example, in Perez, P. et al, J. Exper. Med., 163:166-178 (1986) and Lau, M.A. et al, Proc. Natl. Acad. Sci., USA, 82:8648-8652 (1985), and native, i.e., non-conjugated or non-complexed, antibody, as described in, for example, Herlyn D. et al, Proc. Natl. Acad. Sci., USA, 79:4761 (1982), Schulz, G. et al, Proc. Natl. Acad. Sci., USA, 80:5407 (1983), Capone, P.M. et al, Proc. Natl. Acad. Sci., USA, 80:7328 (1983), Sears, H.F. et al, Cancer Res., 45:5910 (1985), Nepom, G.T. et al, Proc. Natl. Acad. Sci., USA, 81:2864 (1984), Koprowski, H. et al, Proc. Natl. Acad. Sci., USA, 81:216 (1984) and Houghton, A.N. et al, Proc. Natl. Acad. Sci., USA, 82:1242 (1985).

In this embodiment of the invention, the monoclonal antibody-therapeutic agent can be delivered directly to the tumor site thereby directly exposing the tumor tissue to the therapeutic agent.

The following examples are provided for illustrative purposes only.

Example 1

Production of Monoclonal Antibody TURP-27

(A) Production of Hybridomas

Three female BALB/c mice (Harlan Sprague-Dawley, Indianapolis, IN, 20-week-old) were hyperimmunized against a crude membrane antigen preparation derived from a pool of three benign prostatic hyperplasia (BPH) specimens and one prostate adenocarcinoma transurethral resection specimen.

The crude membrane antigen preparation was prepared by incubating single cell suspensions for 1 to 2 hours in hypotonic buffer comprising 20 mM Hepes (pH 7.1), 5.0 mM NaCl, 1.0 mM $MgCl_2$, 0.1 M NaF and 2.0 mM PMSF. The cells were then lysed by Dounce homogenization. The nuclear fraction was removed by centrifugation at 200 x g for 5 minutes, and the supernatant was decanted and recentrifuged at 25,000 x g for 30 minutes at 4°C. The resulting pellet was then redissolved in 1.5 ml of a 37% (v/v) sucrose solution in membrane buffer comprising 20 mM Hepes (pH 6.8), 40 mM NaCl, 0.1 mM EDTA and 2.0 mM PMSF, overlaid with 3.0 ml of a 20% (v/v) sucrose solution in membrane buffer and centrifuged at 2,000 rpm for 16 hours at 2°C. The membrane rich (20-37%) interface was then removed in approximately 1.0 ml, diluted with 10 ml of membrane buffer and centrifuged at 40,000 rpm for 2 hours at 10°C to obtain the crude membrane antigen preparation.

The mice were immunized (day 0) subcutaneously (0.1 ml) and intramuscularly (0.05 ml in rear flanks)

with 100µg of the crude membrane antigen preparation mixed with an equal volume of complete Freund's adjuvant. The mice were boosted with intraperitoneal injections of 100 µg of the crude membrane antigen preparation in 0.1 ml PBS on days 6 and 36. On day 66, the mice were given an intravenous injection of 100 µg of the crude membrane antigen preparation in 0.1 ml PBS and the spleens were removed for fusion four days later. Spleen cells from the hyperimmunized mice were fused with P3-NS-1-Ag4-1 mouse myeloma cells as described by Kohler, G. et al, in Eur. J. Immunol., 6:292-295 (1976).

in the presence of 50% polyethylene glycol (American Type Culture Collection, 1300-1600 MW) according to procedures established by Koprowski, H. et al, Proc. Natl. Acad. Sci. USA, 74:2985-2988 (1977).

After fusion, the cells ($3.0 \times 10^8$ spleen and $3.0 \times 10^7$ myeloma) were washed and resuspended in 300 ml of HSI LoSM medium (Hybridoma Sciences, Inc., Atlanta, Georgia) medium containing 10% (v/v) fetal bovine serum (Hyclone Laboratories, Logan, Utah), $1.0 \times 10^{-4}$ M hypoxanthine, $4.0 \times 10^{-7}$ M aminopterin, $6.4 \times 10^{-5}$ M thymidine, and 50 ug/ml gentamicin (Grand Island Biological Co., Grand Island, New York). The cells were then dispersed in 96-well microtiter plates (Costar, Cambridge, Massachusetts) in 0.2 ml aliquots. Hybridomas that were determined to be of interest, i.e., that produced monoclonal antibodies having binding specificity to prostate antigens but not to non-prostate antigens, were doubly cloned by limiting dilution. In this manner, two hybridomas producing monoclonal antibodies designated as TURP-27 and TURP-73 were developed. TURP-27 and TURP-73 have been deposited at the American Type Culture Collection, Rockville, Maryland under ATCC Nos. 40292 and 40293, respectively.

(B) Screening of Hybridoma Supernatants

Supernatants from actively growing hybridomas were screened for binding activity against a number of normal, hyperplastic, virally-transformed, and malignant cell lines (Starling, J.J. et al, Cancer Res., 42:3084-3089 (1982)), and crude membrane extracts using a solid-phase RIA as described by Starling, J.J. et al, J. Supramol. Struct. 11:563-577 (1979).

The monoclonal antibodies were used as culture supernatants from hybridomas grown in the presence of 10% (v/v) fetal calf serum under standard culture conditions. The protein concentration was determined by the method of Lowry, O.H. et al, J. Biol. Chem. 193:265-275 (1951).

The culture supernatants were found to contain approximately 15 to 30 µg monoclonal antibody/ml.

Whole cells or crude membrane extracts were attached by glutaraldehyde fixation to individual wells of 96-well polyvinylchloride microtiter plates (Dynatech Laboratories, Inc., Alexandria, Virginia). More specifically, whole cells or crude membrane extracts 3.0 µg/well were added to each well and the wells were dried. Then, 0.1% (v/v) glutaraldehyde was added to each well for a few minutes and washed with PBS. The fixed cells were incubated with the hybridoma culture supernatants for 1 hour at room temperature. After extensive washing, 25 ml of affinity-purified (Guimezanes, A. et al, Eur. J. Immun., 6:69-72 (1976)), $^{125}$I-labelled (Hunter, R., Proc. Soc. Biol. Med., 133:989-992 (1970)), F(ab')$_2$ fragments (Fridman, W.H. et al, Cell Immun., 11:442-445 (1974)), of rabbit anti-mouse IgG serum (Miles Laboratories, Elkhardt, Indiana) were added to each well (100,000 cpm) for 1 hour. The plate was washed, and individual wells were cut out of the flexible microtiter plate and were counted in a gamma counter. The results are shown in Tables 1 and 2 below.

References to TURP 73 in the accompanying are by way of comparative example and are outside the scope of the present application.

## TABLE 1

Binding of Monoclonal Antibodies TURP-27
and TURP-73 to Human Cell Lines in Solid-Phase RIA

| Cell Lines | Origin | Binding Ratio 27 73[a] (Comparative Example) | |
|---|---|---|---|
| Prostate Carcinoma | brain metastasis | 1.6 | 4.0 |
| DU145 | brain metastasis | 1.6 | 4.0 |
| PC-3 | bone metastasis | 2.1 | 5.8 |
| LNCaP | lymph node metastasis | 0.9 | 3.4 |
| Bladder Carcinoma | | | |
| 253 J | lymph node metastasis | 1.2 | 1.5 |
| T-24 | primary | 1.5 | 2.1 |
| 639V | primary | 1.6 | 0.9 |
| Melanoma | | | |
| WM-9 | lymph node metastasis | 1.0 | 1.3 |
| H1477 | primary | 1.2 | 2.1 |
| Pancreatic Carcinoma | | | |
| MIA | primary | 1.5 | 2.3 |
| Panc-1 | primary | 0.9 | 3.0 |
| Kidney Carcinoma | | | |
| A498 | primary | 1.5 | 2.3 |
| CaKi-2 | primary | 1.3 | 1.9 |
| Breast Carcinoma | | | |
| ZR-75-1 | ascites | 1.3 | 7.0 |
| BT-20 | primary | 0.7 | 1.8 |
| Colorectal Carcinoma | | | |
| SE 1116 | primary | 1.0 | 8.9 |
| CX-1 | primary | 1.3 | 11.4 |
| Leukemia | | | |
| MOLT-3 | T cell | 1.2 | 1.9 |
| CCRF-SB | B cell | 1.5 | 1.5 |

## TABLE 1 (cont'd)

| Cell Lines | Origin | Binding Ratio | |
|---|---|---|---|
| | | 27 | 73 [a] |
| | | (Comparative Example) | Example |
| **Lymphoma** | | | |
| Raji | Burkitt | 2.1 | 1.8 |
| U937 | histocytic | 1.9 | 1.9 |
| **Virally-transformed** | | | |
| CMV-Mj-HEL-1 | cytomegalovirus | 0.9 | 1.0 |
| **Normal fibroblasts** | | | |
| WI-38 | lung | 1.1 | 1.2 |
| Flow 4000 | kidney | 2.0 | 2.5 |
| GM-10 | skin | 1.0 | 1.6 |

a

Binding ratio represents the total number of cell-bound counts using hybridoma culture supernatant divided by the number of cell-bound counts using the P3X63/Ag8 myeloma (ATCC No. TIB 9) supernatant which contains the MOPC 21 $IgG_1$. Reactions are scored positive if the binding ratio is greater than 3. The results are the average of duplicate determinations with less than 20% variance between wells for any one test.

TABLE 2

Binding of Monoclonal Antibodies TURP-27 and TURP-73 To
Human Tissue Membrane Preparations in a Solid-Phase RIA

| Specimen[b] | Tissue Type | Binding Ratio[a] 27 | 73 (comparative example) |
|---|---|---|---|
| Ca 1046 | prostate carcinoma | 6.4 | 5.7 |
| Ca 1124 | prostate carcinoma | 8.4 | 22.5 |
| Ca 1126 | prostate carcinoma | 9.6 | 18.4 |
| Ca 1129 | prostate carcinoma | 4.0 | 13.8 |
| Ca 1122 | BPH | 17.4 | 6.6 |
| P-5-80 | BPH | 5.7 | 25.8 |
| P-125-80 | BPH | 1.4 | 8.0 |
| P-144-80 | BPH | 11.4 | 19.3 |
| P-151-80 | BPH | 21.1 | 55.2 |
| P-8-80 | normal prostate | 1.0 | 7.1 |
| P-16-80 | normal prostate | 1.8 | 10.5 |
| P-104-80 | normal prostate | 1.3 | 6.7 |
| P-108-80 | normal prostate | 1.6 | 7.7 |
| P-110-80 | normal prostate | 1.9 | 13.8 |
| P-117-80 | normal prostate | 4.0 | 9.1 |
| P-119-80 | normal prostate | 1.0 | 9.1 |
| TURP[c] | prostate carcinoma, BPH | 4.3 | 13.0 |
| Ca 1101 | normal bladder | 1.4 | 2.8 |
| Ca 1037 | normal bladder | 1.7 | 1.9 |
| Ca 1037 | normal ovary | 1.3 | 1.1 |
| Ca 1030 | normal ovary | 1.0 | 1.0 |
| Ca 1005 | normal colon | 2.1 | 1.6 |
| Ca 1030 | normal colon | 1.8 | 1.3 |
| Ca 1037 | normal kidney | 1.9 | 10.8 |
| Ca 1042 | normal kidney | 1.8 | 11.5 |
| Ca 1013 | normal liver | 2.5 | 1.6 |
| Ca 1026 | normal liver | 1.7 | 1.2 |
| Ca 1030 | normal lymph node | 2.2 | 1.4 |
| Ca 1030 | normal pancreas | 2.3 | 2.8 |
| Ca 1008 | normal spleen | 2.1 | 1.7 |
| Ca 1028 | normal spleen | 2.3 | 1.8 |
| Ca 1028 | normal testicle | 1.8 | 1.1 |
| Ca 1043 | normal testicle | 2.1 | 1.7 |
| Ca 1031 | normal ureter | 2.4 | 1.6 |
| Ca 1037 | normal breast | 3.0 | 1.9 |

[a]  See footnote "a" in Table 1.

[b]  Surgical and autopsy specimens were assigned
identification numbers to maintain confidentiality.
Specimens were stored at -70°C until utilized for
crude membrane preparations.

[c]  Pooled BPH and prostrate carcinoma specimens were
used as immunogen for hybridoma production.

As the results in Tables 1 and 2 above demonstrate, the monoclonal antibody designated as TURP-27
was not reactive against any of the human cell lines tested (Table 1) but did bind to four of four prostate
adenocarcinomas, four of five BPH and one of seven normal prostate membrane preparations (Table 2).
TURP-27 also did not react with any other normal tissues examined in the RIA including bladder, ovary,
colon, kidney, liver, lymph node, pancreas, spleen, testicle, ureter, and breast (Table 2).

On the other hand, the monoclonal antibody designated as TURP-73 exhibited a reactivity to a wider range of cell lines and tissues. Specifically, TURP-73 bound to the three well-characterized human prostate adenocarcinoma cell lines (DU145, PC-3, LNCaP) as well as to the ZR-75-1 breast carcinoma and the SW1116 and CX-1 colorectal carcinoma cell lines (Table 1). TURP-73 also reacted strongly to all normal, benign hyperplastic, and malignant prostate membrane preparations tested and also bound to normal kidney (Table 2).

## (C) Immunofluorescent Staining

Immunofluorescence of live and fixed cells was performed as described in Starling, J.J. et al, Cancer Res., 42:3084-3089 (1982) and Starling, J.J. et al, Cancer Res., 45:804-808 (1985).

More specifically, $10^6$ cells were placed in plastic tubes and washed twice with cold PBS. The cell pellets were resuspended in 100 $\mu$l of hybridoma culture supernatant and incubated for 30 minutes at 4°C. The cell pellets were washed twice with cold PBS and then mixed with 25 ml of a 1:20 dilution of fluorescein isothiocyanate-conjugated anti-mouse IgG (heavy and light chain specific; Cappel Laboratories, Cochranville, Pennsylvania) and incubated for 30 minutes at 4°C. The cells were washed twice more in cold PBS and stored on ice until ready to view. Wet mounts of cell suspensions were made as soon as possible and examined with a Zeiss fluorescent microscope. The number of cells showing positive fluorescence out of 300 total cells were counted to determine the percentage of the cells exhibiting fluorescence.

TURP-27 was not found to bind by immunofluorescence, to live or methanol:acetic acid-, formalin- or ethanol-fixed DU145 or PC-3 cells, confirming the non-reactivity of this monoclonal antibody to prostate tumor cell lines which was observed by RIA. On the other hand, TURP-73 was found to provide a strong membrane fluorescence of PC-3 cells that persisted following alcohol or formalin fixation.

## (D) Quantitative Absorption

Monoclonal antibody absorption analyses utilizing crude membrane antigen preparations or whole cells were performed as described in Starling, J.J. et al, Cancer Res., 42:3084-3089 (1982), and protein determinations were carried out by the method of Lowry, O.H. et al, J. Biol. Chem., 193:265-275 (1951). Dilutions (1:10 and 1:500) of culture supernatants from TURP-27 and TURP-73 were used, respectively. Varying amounts of crude membrane antigen preparations were added and the final volume was 0.11 ml. The assay was performed as described by Wright, G.L. et al, Urol., 19:351-355 (1982).

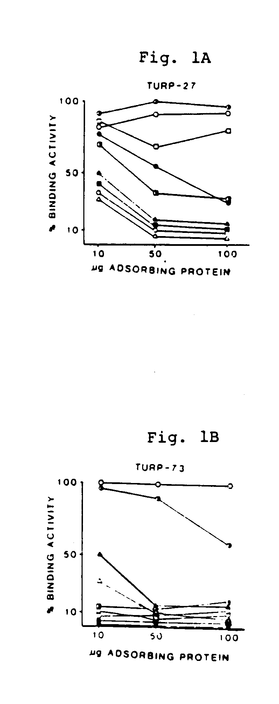

The results are shown in Figures 1A and 1B.

In Figures 1A and 1B, ● represents Ca 1124 prostate adenocarcinoma; ◐ represents Ca 1126 prostate adenocarcinoma; ▲ represents P-144-80 benign prostatic hyperplasia; △ represents P-151-80 benign prostatic hyperplasia; o represents Ca-1122 benign prostatic hyperplasia: □ represents P-16-80 normal prostate; ■ represents P-117-80 normal prostate; ○ represents Ca 1026 normal liver; and ◑ represents Ca 1005 normal colon. In Figures 1A and 1B, the percentage of binding activity is defined as follows:

$$100 \ \times \ \frac{(a-b)}{(c-b)}$$

wherein a is the cpm of the absorbed monoclonal antibody; b is the cpm of P3X63/Ag8 myeloma MOPC 21 IgG1 control monoclonal antibody and c is the cpm of the unadsorbed monoclonal antibody. The target antigen employed was Ca 1124 prostate adenocarcinoma membrane preparation.

Figures 1A and 1B demonstrate that TURP-27 was not reactive with tissues which had no apparent binding activity in the RIA (normal liver, normal colon, normal prostate P16-80) while tissues that did react in the RIA (Table 2) also demonstrated significant absorption activity for TURP-27. TURP-73 exhibited a similar pattern although some absorption was observed with high levels of a normal colon membrane extract (Ca 1005) which was not reactive with TURP-73 in the RIA (Table 2).

It was also found that absorption of TURP-27 with $10^7$ DU145, PC-3 or LNCaP cells or 100 $\mu$g of cell protein extracts of these prostate tumor lines failed to reduce the binding of this monoclonal antibody to the Ca 1126 prostate carcinoma target antigen; whereas, it was found that 50-85% of the binding of TURP-73 to the target antigen was reduced by either whole cells or cell lysates.

## (E) Immunohistochemistry

EP 0 228 243 B1

The binding specificity of TURP-27 and TURP-73 was also evaluated using an immunoperoxidase assay. Avidin-biotin immunoperoxidase analyses were performed on formalin-fixed, paraffin-embedded tissues using the ABC Vectastin kit (Vector Laboratories, Burlingame, California) as described by Wright, G.L. et al, Cancer Res., 43:5509-5516 (1983). The results are set forth in Table 3 below.

TABLE 3

| Immunoperoxidase Staining of Normal, Benign Hyperplastic And Cancerous Human Tissue With Monoclonal Antibodies TURP-27 and TURP-73[a] | | | | |
|---|---|---|---|---|
| Tissue Source | Tissue Type | Monoclonal Antibodies | | |
| | | 27 | 73 (comparative example) | |
| Prostate | Primary carcinoma | 11/11 | 11/11 | |
| | Metastatic carinoma[b] | 6/6 | 3/5 | |
| | BPH | 6/6 | 6/6 | |
| | Normal[c] | 3/3 | 3/3 | |
| Bladder | Primary carcinoma | 0/6 | 0/6 | |
| | Normal | 0/6 | 0/6 | |
| Breast | Primary carcinoma | 0/6 | 0/6 | |
| | Normal | 3/6[d] | 0/6 | |
| Colon | Primary carcinoma | 0/6 | 2/6 | |
| | Normal | 0/6 | 0/6 | |
| Kidney | Primary carcinoma | 0/6 | 0/6 | |
| | Normal | 0/6 | 5/6[e] | |
| Lung | Primary carcinoma | 0/6 | 0/6 | |
| | Normal | 0/6 | 0/6 | |
| Pancreas | Primary carinoma | 0/6 | 0/6 | |
| | Normal | 0/6 | 0/6 | |
| Liver | Normal | 0/4 | 0/4 | |

[a] Serial sections of formalin-fixed tissue were reacted with approximately 3.0 to 6.0 $\mu$g of antibody per slide.
[b] Panel consisted of five prostate adenocarcinoma lymph node metastases and one lung metastasis.
[c] Specimens obtained from young males between the ages of 18-23 with no evidence of disease based on histopathological examination. TURP-27 was reactive with a few cells in less than 10% of the glandular elements within the sections.
[d] Several cells were stained within normal ducts.
[e] Proximal tubules were weakly stained in 5/6 specimens. Renal casts present in three specimens were strongly stained.

As the results in Table 3 above demonstrate, TURP-27 reacted with all prostate specimens and did not bind to normal or malignant tissues derived from bladder, colon, kidney, lung, or pancreas. TURP-27 was not reactive with six breast carcinoma specimens but did react to three of six normal breast tissue samples, although the reactivity was confined to a few cells lining some of the normal ducts. However, as discussed above, quantitative adsorption analyses failed to detect binding by the TURP-27 monoclonal antibody to these normal breast tissues. Monoclonal antibody TURP-73 also reacted with normal, benign hyperplastic, and malignant prostate tissues in the immunoperoxidase assay and to two of six colon carcinomas. Weak staining of the proximal tubules and renal casts in normal kidney specimens was also observed with TURP-73.

(F) Binding of Monoclonal Antibodies to PAP and PSA

1. Direct Binding Solid-Phase RIA

12

The binding activity of purified TURP-27 and TURP-73 to purified human PAP and PSA was evaluated using a direct binding solid-phase RIA against 30 ng of PAP and 150 ng of PSA dried and fixed to individual wells of polyvinylchloride microtiter plates as described above.

TURP-27 and TURP-73 were purified to 95% homogeneity from hybridomas grown in serum-free medium (HSI LoSM, supplemented with ITS premix, Collaborative Research, Inc., Lexington, Massachusetts) as described previously by Wright, G.L. et al. Cancer Res., 43:5509-5516 (1983), by Protein A-Sepharose (Sigma Chemical Co., St. Louis, Missouri) affinity chromatography for TURP-73 ($IgG_{2a}$) and by precipitating the TURP-27 monoclonal antibody ($IgG_3$) by dialysis against 5.0 mM Tris-HCl, pH 7.2 and reconstitution in PBS according to the methods disclosed by J.J. Langone in J. Immunol. Meth., 55:277-296 (1982) and J.W. Goding in J. Immunol. Meth., 39:285-308(1980).

The degree of purification was determined by polyacrylamide gel electrophoresis and qualitatively evaluated. The serum-free preparations were concentrated to 1 to 2.3 mg monoclonal antibody/ml.

Prostate-specific antigen (PSA) was purified from human seminal plasma according to the methods described by Wang, M.C. et al, Oncol., 39:1-5 (1982), and is commercially available from Hybritech, Inc., La Jolla, California. Prostatic acid phosphatase is commercially available from Sigma Chemical Co.

Monoclonal antibodies to PAP and PSA were used as positive controls while MOPC 21 $IgG_1$ was used as a negative control. Monoclonal antibodies to PAP and PSA are commercially available from Hybritech, Inc., La Jolla, California. The results are shown in Table 4 below.

TABLE 4

| Reactivity of Monoclonals TURP-27 and TURP-73 To Prostatic Acid Phosphatase (PAP) and Prostate-Specific Antigen (PSA) In A Direct Binding RIA | | |
|---|---|---|
| Antibody[a] | Target | CPM Bound |
| Anti-PAP | PAP | 1450 |
| TURP-27 | PAP | 210 |
| TURP-73 | PAP | 165 |
| MOPC 21 | PAP | 300 |
| Anti-PSA | PSA | 16590 |
| TURP-27 | PSA | 408 |
| TURP-73 | PSA | 210 |
| MOPC 21 | PSA | 320 |

[a] Antibodies were used at concentrations of 7.0 $\mu$g/ml.

2. Blocking Radioimmunoassay

A blocking radioimmunoassay was also performed by incubating 11 $\mu$l of a 20 $\mu$g/ml concentration of each monoclonal antibody (2.0 $\mu$g/ml) in 0.11 ml final volume of PBS containing 10% (v/v) gamma globulin-free horse serum in the absence or presence of 300 ng purified PAP or 4.0 $\mu$g PSA. The human prostate adenocarcinoma membrane crude extract Ca 1126 was used at 3.0 $\mu$g/well as the target antigen. The results are shown in Table 5 below.

TABLE 5

| Inhibition of Binding of TURP-27 and TURP-73 To Their Target Antigens By Purified Prostatic Acid Phosphatase (PAP) and Prostate-Specific Antigen (PSA) | | |
|---|---|---|
| Antibody | Blocking Antigen | CPM Bound |
| Anti-PAP | None | 5400 |
| Anti-PAP | PAP | 450 |
| Anti-PAP | Ca 1126[a] | 178 |
| Anti-PSA | None | 8400 |
| Anti-PSA | PSA | 1450 |
| Anti-PSA | Ca 1126 | 1204 |
| TURP-27 | None | 4200 |
| TURP-27 | PAP | 4300 |
| TURP-27 | PSA | 4000 |
| TURP-27 | Ca 1126 | 270 |
| TURP-73 | None | 8700 |
| TURP-73 | PAP | 8800 |
| TURP-73 | PSA | 7800 |
| TURP-73 | Ca 1126 | 100 |

[a] Crude membrane extract (100 $\mu$g).

The results in Tables 4 and 5 above demonstrate that TURP-27 and TURP-73 did not bind to PAP or PSA which was absorbed and fixed to a microtiter plate in a direct binding RIA (Table 4) nor did incubation of TURP-27 and TURP-73 with PAP or PSA diminish TURP-27 or TURP-73 binding to the target antigen in a blocking assay (Table 5).

3. Competitive Binding Assay

Competitive binding analysis was also performed by labeling 140 $\mu$g of purified monoclonal antibody TURP-27 and TURP-73 with 0.5 mCi Na $^{125}$I (ICN, Irvine, California) to a specific activity of 2.5 x 10$^6$ cpm/$\mu$g for TURP-27 and 3.0 x 10$^6$ cpm/$\mu$g for TURP-73 using the Iodobead (Pierce Chemical Co., Rockford, Illinois) method as described by Markwell, M.A.K., Anal. Biochem., 125:427-432 (1982).

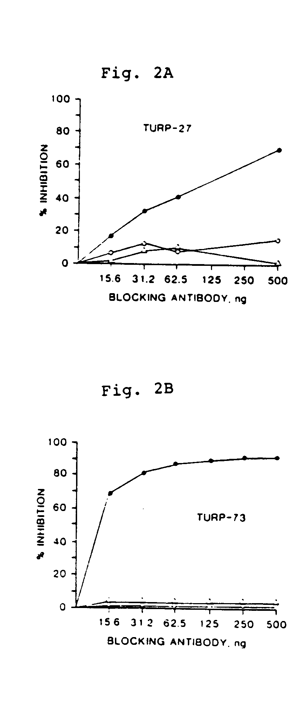

Varying amounts of non-radioactive monoclonal antibodies were allowed to bind to the target antigen, i.e., Ca 1126 prostate adenocarcinoma crude membrane preparation, for 1 hour at room temperature. After washing away unbound monoclonal antibody , 250,000 cpm of $^{125}$I-labelled monoclonal antibody was added for 1 hour at room temperature. Thereafter, the plate was washed and individual wells were counted in a gamma counter. The results are shown in Figures 2A and 2B.

In Figures 2A and 2B, o represents inhibition of binding by monoclonal antibodies specific to PSA; $\Delta$ represents inhibition of binding by monoclonal antibodies specific to PAP and $\bullet$ represents inhibition of binding by TURP-27 (Figure 2A) or TURP-73 (Figure 2B). In Figures 2A and 2B, the percentage of inhibition is defined as follows:

$$100 \text{ x } \left[ 1 - \frac{(a-b)}{(c-b)} \right]$$

wherein a is the cpm of bound $^{125}$I-labelled TURP-27 or $^{125}$I-labelled TURP-73 to Ca 1126 target antigen in the presence of blocking antibodies; b is the cpm of bound $^{125}$I-labelled TURP-27 or $^{125}$I-labelled TURP-73 to a PBS control well containing no target antigen; and c is the cpm of bound $^{125}$I-labelled TURP-27 or $^{125}$I-labelledTURP-73 in the absence of blocking antibodies.

The results in Figures 2A and 2B demonstrate that monoclonal antibodies against PSA or PAP could not effectively block the binding of $^{125}$I-labelled TURP-27 or $^{125}$I-labelled TURP-73 to the Ca 1126 membrane target while unlabelled TURP-27 and TURP-73 were very efficient at blocking radioactive TURP-27 and TURP-73 binding, respectively.

14

(G) Reciprocal Binding Assay

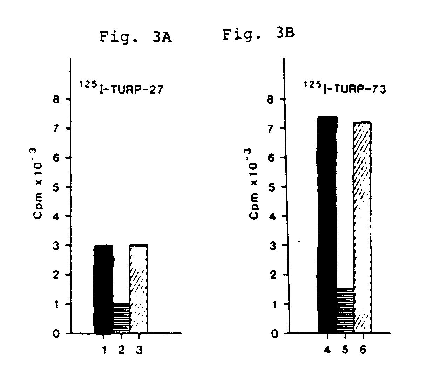

Purified TURP-27 and TURP-73 were labeled with $^{125}$I as described above. A series of two-fold dilutions of unlabeled monoclonal antibodies were added to the target antigen (3.0 $\mu$g, Ca 1126) and incubated for 1 hour at room temperature. After washing, 1 x 10$^6$ cpm of labeled TURP-27 or TURP-73 was added to each well and incubated for another hour at room temperature. The wells were washed and the radioactivity was counted in a gamma counter. The results are shown in Figures 3A and 3B.

In Figure 3A, 1 represents without addition of unlabelled antibodies; 2 represents after addition of unlabelled TURP-27; and 3 represents after addition of unlabelled TURP-73. In Figure 3B, 4 represents without addition of unlabelled antibodies; 5 represents after addition of unlabelled TURP-73; and 6 represents after addition of unlabelled TURF-27.

Figures 3A and 3B demonstrate that binding of the labeled monoclonal antibody was inhibited only by unlabeled antibodies from the autologous clone. These data demonstrate that TURP-27 and TURP-73 detect distinct antigenic determinants.

(H) Isotyping of Monoclonal Antibodies

Antibody heavy-chain and light-chain classes were determined using a mouse immunoglobulin subtype identification kit available from Boehringer-Mannheim Biochemicals, Indianapolis, Indiana, TURP-27 was determined to be of the isotype IgG$_3$,K and TURP-73 of the isotype IgG$_{2a}$,K.

(I) Summary of Results

TURP-27 was not reactive to any of the human cell lines tested (Table 1) but did react to BPH and prostate adenocarcinoma tissue specimens (Tables 2 and 3). Reactivity was also observed to one of seven normal prostate membrane preparations. The prostate-specific binding activity of TURP-27 in the RIA (Table 2) was also seen in the immunoperoxidase assay except for three normal breast specimens which contained several stained cells within a few normal ducts (Table 3). Two of these breast specimens were available as frozen tissue and were utilized to make crude membrane preparations. Monoclonal antibody TURP-27 was not reactive with these specimens in the solid-phase RIA nor was any absorption activity observed with either of these tissues at concentrations as high as 100 $\mu$g absorbing protein. Substantial absorption activity was observed, however, by TURP-27-reactive samples at protein levels much lower than 100 $\mu$g (Figure 1A).

Monoclonal antibody TURP-73 bound strongly to normal, BPH, and malignant prostate tissue as well as to colorectal carcinoma, breast carcinoma, and normal kidney specimens (Tables 1, 2 and 3). This monoclonal antibody was not widely cross-reactive to non-prostate normal or malignant cells but its antigen was present on a wider array of cells types and tissues than the TURP-27 antigen.

TURP-27 bound to one of six normal prostate membrane preparations by solid-phase RIA (Table 2). The six unreactive normal prostate specimens were from young men aged 18 to 28, while the one reactive sample was from a 66-year-old male. Due to the high incidence in prostate of occult hyperplasia and neoplasia for men at this age (Starling, J.J. et al, Monoclonal Antibodies and Cancer, pp. 253-286 (1984), New York, Marcel Dekker), it is possible that this specimen was contaminated with cells that were not normal. This apparent low reactivity to normal prostate by TURP-27 was also examined by quantitative adsorption analysis (Figure 1A). This technique suggested that the TURP-27 antigen was in normal prostate tissue but at a much lower concentration than in BPH and prostate adenocarcinoma (Table 2 and Figure 1A). Of the normal prostate specimens examined in the immunoperoxidase assay (Table 3), only 10% of the ductal epithelial cells were reactive with the TURP-27 monoclonal antibody. This result was consistent with the low binding activity of TURP-27 in the solid-phase RIA and quantitative adsorption assay for these specimens. Collectively, the data demonstrates that the TURP-27 antigen should be a useful marker for benign and/or malignant growth.

(J) TURP-27 vs Known Prostate Antibodies

Comparison of TURP-27 with other monoclonal antibodies derived against prostate antigens demonstrates that TURP-27 has defined new prostate organ- and tumor-associated antigens. The serological activity of monoclonal antibodies D83.21 (Starling, J.J. et al, Cancer Res., 42:3084-3089 (1982) and Starling, J.J. et al, Monoclonal Antibodies and Cancer, pp. 253-286 (1984), New York, Marcel Dekker), and P6.2 (Wright, G.L. et al, Cancer Res., 43:5509-5516 (1983) and Starling, J.J. et al, Monoclonal Antibodies and Cancer, pp. 253-286 (1984), New York, Marcel Dekker)), derived against the DU145 cell line, described by Mickey, D.D. et al, Prog. Clin. Biol. Res., 37:67-84 (1980), and the PC-3 cell line, described by Kaighn, M.E. et al, Prog. Clin. Biol. Res., 37:85-109 (1980), respectively, quite distinct from TURP-27 in that TURP-27 does not bind to any cell line tested (Table 1). Furthermore, in a limited study neither D83.21 nor P6.2 blocked the binding of TURP-27 to its respective targets.

Monoclonal antibody Pro-3 described by Ware, J.L. et al, Cancer Res., 42:1215-1222 (1982), produced against the PC-3 cell line, binds strongly to the immunizing cell line but not to DU145 prostate tumor cells and reacts much more strongly with prostate adenocarcinoma than with BPH tissues. TURP-27, on the other hand, does not bind to either the DU145 or the PC-3 cell line (Table 1). TURP-27 also reacts strongly with both BPH and malignant prostate tissue (Tables 2 and 3 and Figures 2A).

Monoclonal antibodies 35 and 24 have been described by Frankel, A.E. et al, Proc. Natl. Acad. Sci. USA, 99:903-907 (1982).

These monoclonal antibodies are different from TURP-27 in that monoclonal antibody 24 reacts with the PC-3 cell line and also binds more strongly to normal prostate than to malignant prostate tissue, Frankel, A.E. et al, Proc. Natl. Acad. Sci. USA, 99:903-907 (1982).

On the other hand, TURP-27 does not bind to PC-3 cells (Table 1) and this monoclonal antibody reacts more strongly with prostate adenocarcinoma than to normal prostate (Tables 2 and 4 and Figure 2A).

A monoclonal antibody, KR-P8, directed against the PC-3 prostate tumor cell line has been described by Raynor, R.H. et al, J. Natl. Cancer Inst., 73:617-625 (1984).

The KR-P8 antigen was stated to be a new prostate-specific marker even though extensive binding studies of the monoclonal antibody to a large panel of human normal and tumor tissues was not performed. TURP-27 does not appear to be directed against the KR-P8 antigen because TURP-27 does not react with PC-3 cells (Table 1).

Three monoclonal antibodies produced to the PC-3 immunogen have been described by Carroll, A.M. et al, Clin. Immunol. Immunopath., 33:268-281 (1984).

All three monoclonals bind to the PC-3 targets whereas TURP-27 does not bind to any tumor cell line tested (Table 1).

A panel of eight monoclonal antibodies directed against human prostate cancer cell lines was recently described by Lindgren, J. et al, Hybridoma, 4:37-45 (1985).

Seven of these monoclonal antibodies did not react strongly with fixed tissues in the immunoperoxidase assay which distinguishes them from TURP-27. The one immunohistochemically reactive monoclonal antibody described in this report bound to a nuclear antigen present in all cells tested which is clearly different from the pattern seen by TURP-27 (Table 3).

A human IgM monoclonal antibody, designated MGH-7, produced by fusing lymphocytes from a regional draining lymph node from a patient with prostate carcinoma with murine myeloma cells, was recently described by Love, D.H. et al, J. Urol., 132:780-785 (1984).

This antibody bound both normal and malignant prostate tissues and in this way is similar to the binding of both TURP-27. What distinguishes TURP-27 from MGH-7 is that TURP-27 does not bind to any human cell lines, whereas, MGH-7 binds to both the LNCaP end PC-3 prostate tumor cell lines but not to the DU145 prostate tumor cell line.

## Claims
## Claims for the following Contracting States : BE, CH, DE, FR, GB, IT, LI, LU, NL, SE

1. A monoclonal antibody having binding specificity to human prostate tumor associated antigens but not having binding specificity to prostate-specific antigen or prostatic acid phosphatase characterised in that the antibody is TURP-27 having the identifying characteristics of ATCC No. 40 292.

2. A monoclonal antibody as claimed in claim 1 for use as a medicament.

3. The use of a monoclonal antibody in the manufacture of a medicament for the diagnosis of prostate tumors or metastases thereof characterised by a monoclonal antibody as claimed in claim 1.

4. A method for diagnosing prostate tumors and metastases thereof comprising the steps of:
   (a) exposing a sample of body fluid to a monoclonal antibody having binding specificity to human prostate tumor-associated antigens but not having binding specificity to prostate-specific antigen or prostatic acid phosphatase as claimed in claim 1;
   (b) determining the amount of monoclonal antibody binding to substances present in the body fluid;

and

(c) comparing the amount of monoclonal antibody bound to body fluid substances to a predetermined base level to ascertain the presence of prostate tumors or metastases thereof.

5. A method as claimed in claim 4 wherein the body fluid is blood, serum, seminal plasma, semen, urine or prostatic fluid.

6. A method as claimed in claim 4 wherein the amount of monoclonal antibody binding to substance present in the body fluid is determined by means of a radioimmunossay or by means of an enzyme immunoassay.

7. The use of a monoclonal antibody as claimed in claim 1 conjugated to a marker in the manufacture of a medicament for diagnosing prostate tumors or metastases thereof.

8. A use as claimed in claim 7 wherein the marker is a nuclear magnetic spin-resonance isotope detectable by a nuclear magnetic imaging device.

9. A use as claimed in claim 8 wherein the marker is gadolinium.

10. A use as claimed in claim 6 wherein the marker is a radioactive substance detectable by a gamma scintillation camera.

11. A use as claimed in claim 10 wherein the radioactive substance is $I^{125}$, $I^{131}$, $I^{123}$, $In^{111}$, $In^{113}$, $Ga^{67}$, $Ga^{68}$, $Ru^{97}$, $Ru^{103}$, $Hg^{197}$, $Hg^{203}$ or $Tc^{99m}$.

12. The use of a monoclonal antibody-therapeutic agent in the manufacture of a medicament for treating prostate tumors or metastases thereof characterised by a monoclonal antibody as claimed in claim 1.

13. A use as claimed in claim 12 wherein the monoclonal antibody-therapeutic agent comprises an antibody coupled to a drug, an antibody coupled to a toxin, an antibody coupled to an isotope, a heterobifunctional antibody or a native antibody.

**Claims for the following Contracting States : AT, ES**

1. A method for the manufacture of a medicament comprising the incorporation of a monoclonal antibody having binding specificity to human prostate tumor associated antigens but not having binding specificity to prostate-specific antigen or prostatic acid phosphatase characterised in that the antibody is TURP-27 having the identifying characteristics of ATCC No. 40 292.

2. A method for the manufacture of a medicament for the diagnosis of prostate tumors and metastases thereof comprising the incorporation of a monoclonal antibody having binding specificity to human prostate tumor associated antigens but not having binding specificity to prostate-specific antigen or prostatic acid phosphatase characterised in that the antibody is TURP-27 having the identifying characteristics of ATCC No. 40 292.

3. A method for diagnosing prostate tumors and metastases thereof comprising the steps of:
a) exposing a sample of body fluid obtained from a patient to a monoclonal antibody TURP-27 having the identifying characteristics of ATCC No. 40 292 having binding specificity to human prostate tumor associated antigens but not having binding specificity to prostate-specific antigen or prostatic acid;
b) determining the amount of monoclonal antibody binding to substance present in the body fluid; and
c) comparing the amount of monoclonal antibody bound to body fluid substance to a predetermined base level to ascertain the presence of prostate tumors or metastases thereof.

4. A method as claimed in claim 3 wherein the body fluid is blood, serum, seminal plasma, semen, urine or prostatic fluid.

5. A method as claimed in claim 4 wherein the amount of monoclonal antibody binding to substance present in the body fluid is determined by means of a radioimmunossay or by means of an enzyme immunoassay.

6. A method for the manufacture of a medicament for diagnosing prostatic tumors or metastases thereof comprising the incorporation of a monoclonal antibody having binding specificity to human prostate tumor associated antigens but not having binding specificity to prostate-specific antigen or prostatic acid phosphatase conjugated to a marker characterised in that the antibody is TURP-27 having the identifying characteristics of ATCC No. 40 292.

7. A method as claimed in claim 6 wherein the marker is a nuclear magnetic spin-resonance isotope detectable by a nuclear magnetic imaging device.

8. A method as claimed in claim 6 wherein the marker is gadolinium.

9. A method as claimed in claim 6 wherein the marker is a radioactive substance detectable by a gamma scintillation camera.

10. A method as claimed in claim 9 wherein the radioactive substance is $I^{125}$, $I^{131}$, $I^{123}$, $In^{111}$, $In^{113}$, $Ga^{67}$, $Ga^{68}$, $Ru^{97}$, $Ru^{103}$, $Hg^{197}$, $Hg^{203}$ or $Tc^{99m}$.

11. A method for the manufacture of a medicament for treating prostatic tumors or metastases thereof comprising the incorporation of a monoclonal antibody-therapeutic agent wherein the monoclonal antibody has binding specificity to human prostate tumor associated antigens but does not have binding specificity to prostate-specific antigen or prostatic acid phosphatase characterised in that the antibody is TURP-27 having the identifying characteristics of ATCC No. 40 292.

12. A method as claimed in claim 11 wherein the monoclonal antibody-therapeutic agent comprises an antibody coupled to a drug, an antibody coupled to a toxin, an antibody coupled to an isotope, a heterobifunctional antibody or a native antibody.

**Claims for the following Contracting State : GR**

1. A method for the preparation of a medicament comprising a monoclonal antibody having binding specificity to human prostate tumor associated antigens but not having binding specificity to prostate-specific antigen or prostatic acid phosphatase characterised in that the antibody is TURP-27 having the identifying characteristics of ATCC No. 40 292.

2. A method for the preparation of a medicament for the diagnosis of prostate tumors and metastases thereof, comprising a monoclonal antibody having binding specificity to human prostate tumor associated antigens but not having binding specificity to prostate specific antigen or prostatic acid phosphatase and characterised in that the antibody is TURP-27 having the identifying characteristics of ATCC No. 40 292.

3. A method for diagnosing prostate tumors and metastases thereof comprising the steps of:
   a) exposing a sample of body fluid to monoclonal antibody TURP-27 having the identifying characteristics of ATCC No. 40 292 having binding specificity to human prostate tumor associated antigens but not having binding specificity to prostate-specific antigen or prostatic acid;
   b) determining the amount of monoclonal antibody binding to substance present in the body fluid; and
   c) comparing the amount of monoclonal antibody bound to body fluid substance to a predetermined base level to ascertain the presence of prostate tumors or metastases thereof.

4. A method as claimed in claim 3 wherein the body fluid is blood, serum, seminal plasma, semen, urine or prostatic fluid.

5. A method as claimed in claim 4 wherein the amount of monoclonal antibody binding to substance present in the body fluid is determined by means of a radioimmunossay or by means of an enzyme immunoassay.

6. A method for the manufacture of a medicament for diagnosing prostatic tumors or metastases thereof incorporating a monoclonal antibody having binding specificity to human prostate tumor associated antigens but not having binding specificity to prostate-specific antigen or prostatic acid phosphatase conjugated to a marker characterised in that the antibody is TURP-27 having the identifying characteristics of ATCC No. 40 292.

7. A method as claimed in claim 6 wherein the marker is a nuclear magnetic spin-resonance isotope detectable by a nuclear magnetic imaging device.

8. A method as claimed in claim 6 wherein the marker is gadolinium.

9. A method as claimed in claim 6 wherein the marker is a radioactive substance detectable by a gamma scintillation camera.

10. A method as claimed in claim 9 wherein the radioactive substance is $I^{125}$, $I^{131}$, $I^{123}$, $In^{111}$, $In^{113}$, $Ga^{67}$, $Ga^{68}$, $Ru^{97}$, $Ru^{103}$, $Hg^{197}$, $Hg^{203}$ or $Tc^{99m}$.

11. A method of manufacturing of a medicament for treating prostatic tumors or metastases thereof, comprising a monoclonal antibody-therapeutic agent wherein the monoclonal antibody has binding specificity to human prostate tumor associated antigens but does not have binding specificity to prostate-specific antigen or prostatic acid phosphatase and characterised in that the antibody is TURP-27 having the identifying characteristics of ATCC No. 40 292

12. A method as claimed in claim 11 wherein the monoclonal antibody-therapeutic agent comprises an antibody coupled to a drug, an antibody coupled to a toxin, an antibody coupled to an isotope, a heterobifuncational antibody or a native antibody.

Revendications
Revendications pour les Etats contractants suivants : BE, CH, DE, FR, GB, IT, LI, LU, NL, SE

1. Anticorps monoclonal ayant une spécificité de liaison aux antigènes associés à la tumeur de prostate humaine, mais n'ayant pas de spécificité de liaison à l'antigène spécifique de la prostate ou de la phosphatase acide prostatique, caractérisé en ce que l'anticorps est TURP-27 ayant les caractéristiques d'identification de ATCC No. 40 292.

2. Anticorps monoclonal selon la revendication 1, à utilisation de médicament.

3. Utilisation d'un anticorps monoclonal dans la fabrication d'un médicament pour le diagnostic des tumeurs de la prostate ou de ses métastases, caractérisée par un anticorps monoclonal selon la revendication 1.

4. Procédé pour le diagnostic des tumeurs de la prostate et ses métastases comprenant les étapes suivantes :
a) exposer un échantillon de fluide corporel à un anticorps monoclonal ayant une spécificité de liaison aux antigènes associés à la tumeur de la prostate humaine mais n'ayant pas de spécificité de liaison à l'antigène spécifique de la prostate ou la phosphatase acide prostatique, selon la revendication 1;
b) déterminer la quantité d'anticorps monoclonal lié aux substances présentes dans le fluide corporel; et
c) comparer la quantité d'anticorps monoclonal lié aux substances du fluide corporel à un niveau de base prédéterminé pour constater la présence de tumeurs de la prostate ou de leurs métastases.

5. Procédé selon la revendication 4, dans lequel le fluide corporel est le sang, le sérum, le plasma séminal, le sperme, l'urine ou le fluide prostatique.

6. Procédé selon la revendication 4, dans lequel la quantité d'anticorps monoclonal liant la substance présente dans le fluide corporel est déterminée au moyen d'un essai radio-immuno ou au moyen d'un essai enzyme immuno.

7. Utilisation d'un anticorps monoclonal selon la revendication 1 conjugué à un marqueur dans la fabrication d'un médicament pour diagnostiquer les tumeurs de la prostate ou leurs métastases.

8. Utilisation selon la revendication 7, dans laquelle le marqueur est un isotope de résonnance de spin magnétique nucléaire détectable par un dispositif enregistreur d'images de résonnance magnétique nucléaire.

9. Utilisation selon la revendication 8, dans laquelle le marqueur est le gadolinum.

10. Utilisation selon la revendication 7, dans laquelle le marqueur est une substance radioactive détectable par une caméra à scintillation gamma.

11. Utilisation selon la revendication 10, dans laquelle la substance radioactive est $I^{125}$, $I^{131}$, $I^{123}$, $In^{111}$, $In^{113}$, $Ga^{67}$, $Ga^{68}$, $Ru^{97}$, $Ru^{103}$, $Hg^{197}$, $Hg^{203}$ ou $Tc^{99m}$.

12. Utilisation d'un agent thérapeutique - anticorps monoclonal dans la fabrication d'un médicament pour le traitement des tumeurs de la prostate ou de ses métastases, caractérisée par un anticorps monoclonal selon la revendication 1.

13. Utilisation selon la revendication 12, dans laquelle l'agent thérapeutique - anticorps monoclonal comprend un anticorps couplé à un médicament, un anticorps couplé à une toxine, un anticorps couplé à un isotope, un anticorps hétérobifonctionnel ou un anticorps naissant.

**Revendications pour les Etats contractants suivants : AT, ES**

1. Procédé pour la fabrication d'un médicament comprenant l'incorporation d'un anticorps monoclonal ayant une spécificité de liaison aux antigènes associés à la tumeur de prostate humaine, mais n'ayant pas de spécificité de liaison à l'antigène spécifique de la prostate ou de la phosphatase acide prostatique, caractérisé en ce que l'anticorps est TURP-27 ayant les caractéristiques d'identification de ATCC No. 40 292.

2. Procédé pour la fabrication d'un médicament pour le diagnostic des tumeurs de la prostate et de ses métastases comprenant l'incorporation d'un anticorps monoclonal ayant une spécificité de liaison aux antigènes associés à la tumeur de prostate humaine, mais n'ayant pas de spécificité de liaison à l'antigène spécifique de la prostate ou de la phosphatase acide prostatique, caractérisé en ce que l'anticorps est TURP-27 ayant les caractéristiques d'identification de ATCC No. 40 292.

3. Procédé pour le diagnostic des tumeurs de la prostate et ses métastases comprenant les étapes suivantes :
a) exposer un échantillon de fluide corporel prélevé sur un patient à un anticorps monoclonal TURP-27 ayant les caractéristiques d'identification de ATCC No. 40 292 ayant une spécificité de liaison aux antigènes associés à la tumeur de la prostate humaine mais n'ayant pas de spécificité de liaison à l'antigène spécifique de la prostate ou à l'acide prostatique;
b) déterminer la quantité d'anticorps monoclonal lié aux substances présentes dans le fluide corporel; et
c) comparer la quantité d'anticorps monoclonal lié à la substance du fluide corporel à un niveau de base prédéterminé pour constater la présence de tumeurs de la prostate ou de leurs métastases.

4. Procédé selon la revendication 3, dans lequel le fluide corporel est le sang, le sérum, le plasma séminal, le sperme, l'urine ou le fluide prostatique.

5. Procédé selon la revendication 4, dans lequel la quantité d'anticorps monoclonal liant la substance présente dans le fluide corporel est déterminée au moyen d'un essai radio-immuno ou au moyen d'un essai enzyme immuno.

**6.** Procédé pour la fabrication d'un médicament pour le diagnostic des tumeurs de la prostate ou de ses métastases comprenant l'incorporation d'un anticorps monoclonal ayant une spécificité de liaison aux antigènes associés à la tumeur de prostate humaine, mais n'ayant pas de spécificité de liaison à l'antigène spécifique de la prostate ou de la phosphatase acide prostatique, conjuqué à un marqueur caractérisé en ce que l'anticorps est TURP-27 ayant les caractéristiques d'identification de ATCC No. 40292.

**7.** Procédé selon la revendication 6, dans lequel le marqueur est un isotope de résonnance de spin magnétique nucléaire détectable par un dispositif enregistreur d'images de résonnance magnétique nucléaire.

**8.** Procédé selon la revendication 7, dans lequel le marqueur est le gadolinum.

**9.** Procédé selon la revendication 6, dans lequel le marqueur est une substance radioactive détectable par une caméra à scintillation gamma.

**10.** Procédé selon la revendication 9, dans lequel la substance radioactive est $I^{125}$, $I^{131}$, $I^{123}$, $In^{111}$, $In^{113}$, $Ga^{67}$, $Ga^{68}$, $Ru^{97}$, $Ru^{103}$, $Hg^{197}$, $Hg^{203}$, ou $Tc^{99m}$.

**11.** Procédé de fabrication d'un médicament pour le traitement des tumeurs de la prostate et de ses métastases comportant l'incorporation d'un agent thérapeutique anticorps monoclonal dans lequel l'anticorps monoclonal a une spécificité de liaison aux antigènes associés à la tumeur de prostate humaine, mais n'ayant pas de spécificité de liaison à l'antigène spécifique de la prostate ou de la phosphatase acide prostatique, caractérisé en ce que l'anticorps est TURP-27 ayant les caractéristiques d'identification de ATCC No. 40292.

**12.** Procédé selon la revendication 11, dans lequel l'agent thérapeutique - anticorps monoclonal comprend un anticorps couplé à un médicament, un anticorps couplé à une toxine, un anticorps couplé à un isotope, un anticorps hétérobifonctionnel ou un anticorps naissant.

**Revendications pour l'Etat contractant suivant : GR**

**1.** Procédé pour la préparation d'un médicament comprenant un anticorps monoclonal ayant une spécificité de liaison aux antigènes associés à la tumeur de prostate humaine, mais n'ayant pas de spécificité de liaison à l'antigène spécifique de la prostate ou de la phosphatase acide prostatique, caractérisé en ce que l'anticorps est TURP-27 ayant les caractéristiques d'identification de ATCC No. 40 292.

**2.** Procédé pour la préparation d'un médicament pour le diagnostic des tumeurs de la prostate et de ses métastases comprenant un anticorps monoclonal ayant une spécificité de liaison aux antigènes associés à la tumeur de prostate humaine, mais n'ayant pas de spécificité de liaison à l'antigène spécifique de la prostate ou de la phosphatase acide prostatique, caractérisé en ce que l'anticorps est TURP-27 ayant les caractéristiques d'identification de ATCC No. 40 292.

**3.** Procédé pour le diagnostic des tumeurs de la prostate et ses métastases comprenant les étapes suivantes :
a) exposer un échantillon de fluide corporel à un anticorps monoclonal TURP-27 ayant les caractéristiques d'identification de ATCC No. 40 292 ayant une spécificité de liaison aux antigènes associés à la tumeur de la prostate humaine mais n'ayant pas de spécificité de liaison à l'antigène spécifique de la prostate ou à l'acide prostatique ;
b) déterminer la quantité d'anticorps monoclonal lié aux substances présentes dans le fluide corporel ; et
c) comparer la quantité d'anticorps monoclonal lié à la substance du fluide corporel à un niveau de base prédéterminé pour constater la présence de tumeurs de la prostate ou de leurs métastases.

**4.** Procédé selon la revendication 3, dans lequel le fluide corporel est le sang, le sérum, le plasma séminal, le sperme, l'urine ou le fluide prostatique.

**5.** Procédé selon la revendication 4, dans lequel la quantité d'anticorps monoclonal liant la substance présente dans le fluide corporel est déterminée au moyen d'un essai radio-immuno ou au moyen d'un essai enzyme immuno.

6. Procédé pour la fabrication d'un médicament pour le diagnostic des tumeurs de la prostate ou de ses métastases comprenant un anticorps monoclonal ayant une spécificité de liaison aux antigènes associés à la tumeur de prostate humaine, mais n'ayant pas de spécificité de liaison à l'antigène spécifique de la prostate ou de la phosphatase acide prostatique, conjuqué à un marqueur caractérisé en ce que l'anticorps est TURP-27 ayant les caractéristiques d'identification de ATCC No. 40 292.

7. Procédé selon la revendication 6, dans lequel le marqueur est un isotope de résonnance de spin magnétique nucléaire détectable par un dispositif enregistreur d'images de résonnance magnétique nucléaire.

8. Procédé selon la revendication 7, dans lequel le marqueur est le gadolinum.

9. Procédé selon la revendication 6, dans lequel le marqueur est une substance radioactive détectable par une caméra à scintillation gamma.

10. Procédé selon la revendication 9, dans lequel la substance radioactive est $I^{125}$, $I^{131}$, $I^{123}$, $In^{111}$, $In^{113}$, $Ga^{67}$, $Ga^{68}$, $Ru^{97}$, $Ru^{103}$, $Hg^{197}$, $Hg^{203}$ ou $Tc^{99m}$.

11. Procédé de fabrication d'un médicament pour le traitement des tumeurs de la prostate et de ses métastases comportant un agent thérapeutique anticorps monoclonal dans lequel l'anticorps monoclonal a une spécificité de liaison aux antigènes associés à la tumeur de prostate humaine, mais n'ayant pas de spécificité de liaison à l'antigène spécifique de la prostate ou de la phosphatase acide prostatique, caractérisé en ce que l'anticorps est TURP-27 ayant les caractéristiques d'identification de ATCC No. 40 292.

12. Procédé selon la revendication 11, dans lequel l'agent thérapeutique - anticorps monoclonal comprend un anticorps couplé à un médicament, un anticorps couplé à une toxine, un anticorps couplé à un isotope, un anticorps hétérobifonctionnel ou un anticorps naissant.

**Patentansprüche**
**Patentansprüche für folgende Vertragsstaaten : BE, CH, DE, FR, GB, IT, LI, LU, NL, SE**

1. Monoklonaler Antikörper mit Bindungs-Spezifität für Human-Prostatatumor begleitende Antigene, aber ohne Bindungs-Spezifität für prostata-spezifisches Antigen oder Prostansäurephosphatase, dadurch gekennzeichnet, daß der Antikörper TURP-27 mit den Kennzeichnungs-Charakteristiken von ATCC Nr. 40 292 ist.

2. Monoklonaler Antikörper nach Anspruch 1 zur Verwendung als ein Medikament.

3. Verwendung eines monoklonalen Antikörpers bei der Herstellung eines Medikaments zur Diagnose von Prostatatumoren oder deren Metastasen gekennzeichnet durch einen monoklonalen Antikörper nach Anspruch 1.

4. Verfahren zur Diagnostizierung von Prostatatumoren und ihren Metastasen, umfassend die folgenden Schritte:
(a) eine Körperfluidprobe wird einem monoklonalen Antikörper gemäß Anspruch 1 ausgesetzt, der Bindungs-Spezifität für Human-Prostatumor begleitende Antigene, aber keine Bindungs-Spezifität für prostata-spezifisches Antigen oder Prostan-säurephosphatase hat;
(b) Bestimmen der Bindungsmenge von monoklonalem Antikörper an in dem Körperfluid vorhandene Substanzen; und
(c) Vergleichen der Menge von monoklonalem Antikörper, die an Körperfluidsubstanzen gebunden ist, mit einem vorbestimmten Grundwert, um das Vorliegen Von Prostatatumoren oder deren Metastasen zu bestimmen.

5. Verfahren nach Anspruch 4, wobei das Körperfluid Blut, Serum, Samenplasma, Samen, Urin oder Prostatafluid ist.

**6.** Verfahren nach Anspruch 4, wobei die Menge von monoklonalem Antikörper, die an eine in dem Körperfluid vorhandene Substanz gebunden ist, durch einen Radio-Immuntest oder einen Enzymimmuntest bestimmt wird.

**7.** Verwendung eines monoklonalen Antikörpers nach Anspruch 1, der an eine Markierungssubstanz gekoppelt ist, zur Herstellung eines Medikaments für die Diagnostizierung von Prostatatumoren oder deren Metastasen.

**8.** Verwendung nach Anspruch 7, wobei die Markierungssubstanz ein Kernspinresonanz-Isotop ist, das mit einer kernmagnetischen Abbildungseinrichtung nachweisbar ist.

**9.** Verwendung nach Anspruch 8, wobei die Markierungssubstanz Gadolinium ist.

**10.** Verwendung nach Anspruch 6, wobei die Markierungssubstanz eine radioaktive Substanz ist, die mit einer Gamma-Szintillationskamera nachweisbar ist.

**11.** Verwendung nach Anspruch 10, wobei die radioaktive Substanz $I^{125}$, $I^{131}$, $I^{123}$, $In^{111}$, $In^{113}$, $Ga^{67}$, $Ga^{68}$, $Ru^{97}$, $Ru^{103}$, $Hg^{197}$, $Hg^{203}$, oder $Tc^{99m}$ ist.

**12.** Verwendung eines Heilmittels mit monoklonalem Antikörper bei der Herstellung eines Medikamments zur Behandlung von Prostatatumoren oder deren Metastasen, gekennzeichnet durch einen monoklonalen Antikörper nach Anspruch 1.

**13.** Verwendung nach Anspruch 12, wobei das Heilmittel mit monoklonalem Antikörper einen an ein Arzneimittel gekoppelten Antikörper, einen an ein Toxin gekoppelten Antikörper, einen an ein Isotop gekoppelten Antikörper, einen bifunktionellen Heteroantikörper oder einen nativen Antikörper aufweist.

**Patentansprüche für folgende Vertragsstaaten : AT, ES**

**1.** Verfahren zur Herstellung eines Medikaments, umfassend den Einbau eines monoklonolen Antikörpers mit Bindungs-Spezifität für Human-Prostatatumor begleitende Antigene, aber ohne Bindungs-Spezifität für prostata-spezifisches Antigen oder Prostansäurephosphatase, dadurch gekennzeichnet, daß der Antikörper TURP-27 mit den Kennzeichnungs-Charakteristiken von ATCC Nr. 40 292 ist.

**2.** Verfahren zur Herstellung eines Medikaments zur Diagnose von Prostatatumoren und deren Metastasen, umfassend den Einbau eines monoklonalen Antikörpers mit Bindungs-Spezifität für Human-Prostatatumor begleitende Antigene, aber ohne Bindungs-Spezifität für prostata-spezifisches Antigen oder Prostansäurephosphatase, dadurch gekennzeichnet, daß der Antikörper TURP-27 mit den Kennzeichnungs-Charakteristiken von ATCC Nr. 40 292 ist.

**3.** Verfahren zur Diagnostizierung von Prostatatumoren und deren Metastasen, umfassend die folgenden Schritte:
(a) eine einem Patienten entnommene Körperfluidprobe wird einem monoklonalen Antikörper TURP-27 mit den Kennzeichnungs-Charakteristiken von ATCC Nr. 40 292 ausgesetzt, der Bindungs-Spezifität für Human-Prostatatumor begleitende Antigene, aber keine Bindungs-Spezifität für prostata-spezifisches Antigen oder Prostansäure hat;
(b) Bestimmen der Menge von monoklonalem Antikörper, die an in dem Körperfluid vorhandene Substanz gebunden ist; und
(c) Vergleichen der Menge von monoklonalem Antikörper, die an Körperfluidsubstanz gebunden ist, mit einem vorbestimmten Grundwert, um das Vorliegen von Prostatatumoren oder deren Metastasen zu bestimmen.

**4.** Verfahren nach Anspruch 3, wobei das Körperfluid Blut, Serum, Samenplasma, Samen, Urin oder Prostatafluid ist.

**5.** Verfahren nach Anspruch 4, wobei die Menge von monoklonalem Antikörper, die an in dem Körperfluid vorhandene Substanz gebunden ist, mit einem Radio-Immuntest oder einem Enzymimmuntest bestimmt wird.

6. Verfahren zur Herstellung eines Medikaments zur Diagnose von Prostatatumoren oder deren Metastasen, umfassend den Einbau eines monoklonalen Antikörpers, der Bindungs-Spezifität für Human-Prostatatumor begleitende Antigene, aber keine Bindungs-Spezifität für prostata-spezifisches Antigen oder Prostansäurephosphatase hat und an eine Markierungssubstanz gekoppelt ist, dadurch gekennzeichnet, daß der Antikörper TURP-27 mit den Kennzeichnungs-Charakteristiken von ATCC Nr. 40 292 ist.

7. Verfahren nach Anspruch 6, wobei die Markierungssubstanz ein Kernspinresonanz-Isotop ist, das mit einer kernmagnetischen Abbildungseinrichtung nachweisbar ist.

8. Verfahren nach Anspruch 6, wobei die Markierungssubstanz Gadolinium ist.

9. Verfahren nach Anspruch 6, wobei die Markierungssubstanz eine radioaktive Substanz ist, die mit einer Gamma-Szintillationskamera nachweisbar ist.

10. Verfahren nach Anspruch 9, wobei die radioaktive Substanz $I^{125}$, $I^{131}$, $I^{123}$, $In^{111}$, $In^{113}$, $Ga^{67}$, $Ga^{68}$, $Ru^{97}$, $Ru^{103}$, $Hg^{197}$, $Hg^{203}$ oder $T^{99m}$ ist.

11. Verfahren zur Herstellung eines Medikaments zur Behandlung von Prostatatumoren oder deren Metastasen, umfassend den Einbau eines Heilmittels mit monoklonalem Antikörper, wobei der monoklonale Antikörper Bindungs-Spezifität für Human-Prostatatumor begleitende Antigene, aber keine Bindungs-Spezifität für prostata-spezifisches Antigen oder Prostansäurephosphatase hat, dadurch gekennzeichnet, daß der Antikörper TURP-27 mit den Kennzeichnungs-Charakteristiken von ATCC Nr. 40 292 ist.

12. Verfahren nach Anspruch 11, wobei das Heilmittel mit monoklonalem Antikörper einen an ein Arzneimittel gekoppelten Antikörper, einen an ein Toxin gekoppelten Antikörper, einen an ein Isotop gekoppelten Antikörper, einen bifunktionellen Heteroantikörper oder einen nativen Antikörper aufweist.

**Patentansprüche für folgenden Vertragsstaat : GR**

1. Verfahren zur Herstellung eines Medikaments, das einen monoklonalen Antikörper aufweist, der Bindungs-Spezifität für Human-Prostatatumor begleitende Antigene, aber keine Bindungs-Spezifität für prostata-spezifisches Antigen oder Prostansäurephosphatase hat, dadurch gekennzeichnet, daß der Antikörper TURP-27 mit den Kennzeichnungs-Charakteristiken von ATCC Nr. 40 292 ist.

2. Verfahren zur Herstellung eines Medikaments zur Diagnose von Prostatatumoren und deren Metastasen, umfassend einen monoklonalen Antikörper mit Bindungs-Spezifität für Human-Prostatatumor begleitende Antigene, aber ohne Bindungs-Spezifität für prostata-spezifisches Antigen oder Prostansäurephosphatase, dadurch gekennzeichnet, daß der Antikörper TURP-27 mit den Kennzeichnungs-Charakteristiken von ATCC Nr. 40 292 ist.

3. Verfahren zur Diagnostizierung von Prostatatumoren und deren Metastasen, umfassend die folgenden Schritte:
   (a) eine Körperfluidprobe wird einem monoklonalen Antikörper TURP-27 mit den Kennzeichnungs-Charakteristiken von ATCC Nr. 40 292 ausgesetzt, der Bindungs-Spezifität für Human-Prostatatumor begleitende Antigene, aber keine Bindungs-Spezifität für prostata-spezifisches Antigen oder Prostansäure hat;
   (b) Bestimmen der Menge von monoklonalem Antikörper, die an in dem Körperfluid vorhandene Substanz gebunden ist; und
   (c) Vergleichen der Menge von monoklonalem Antikörper, die an Körperfluidsubstanz gebunden ist, mit einem vorbestimmten Grundwert, um das Vorliegen von Prostatatumoren oder deren Metastasen zu bestimmen.

4. Verfahren nach Anspruch 3, wobei das Körperfluid Blut, Serum, Samenplasma, Samen, Urin oder Prostatafluid ist.

**5.** Verfahren nach Anspruch 4, wobei die Menge von monoklonalem Antikörper, die an in dem Körperfluid vorhandene Substanz gebunden ist, mit einem Radio-Immuntest oder mit einem Enzymimmuntest bestimmt wird.

**6.** Verfahren zur Herstellung eines Medikaments zur Diagnose von Prostatatumoren oder deren Metastasen unter Einbau eines monoklonalen Antikörpers, der Bindungs-Spezifität für Human-Prostatatumor begleitende Antigene, aber keine Bindungs-Spezifität für prostata-spezifisches Antigen oder Prostansäurephosphatase hat und an eine Markierungssubstanz gekoppelt ist, dadurch gekennzeichnet, daß der Antikörper TURP-27 mit den Kennzeichnungs-Charakteristiken von ATCC Nr. 40 292 ist.

**7.** Verfahren nach Anspruch 6, wobei die Markierungssubstanz ein Kernspinresonanz-Isotop ist, das mit einer kernmagnetischen Abbildungseinrichtung nachweisbar ist.

**8.** Verfahren nach Anspruch 6, wobei die Markierungssubstanz Gadolinium ist.

**9.** Verfahren nach Anspruch 6, wobei die Markierungssubstanz eine radioaktive Substanz ist, die mit einer Gamma-Szintillationskamera nachweisbar ist.

**10.** Verfahren nach Anspruch 9, wobei die radioaktive Substanz $I^{125}$, $I^{131}$, $I^{123}$, $In^{111}$, $In^{113}$, $Ga^{67}$, $Ga^{68}$, $Ru^{97}$, $Ru^{103}$, $Hg^{197}$, $Hg^{203}$ oder $Tc^{99m}$ ist.

**11.** Verfahren zur Herstellung eines Medikaments zur Behandlung von Prostatatumoren oder deren Metastasen, umfassend ein Heilmittel mit einem monoklonalen Antikörper, wobei der monoklonale Antikörper Bindungs-Spezifität für Human-Prostatatumor begleitende Antigene, aber keine Bindungs-Spezifität für prostata-spezifisches Antigen oder Prostansäurephosphatase hat, dadurch gekennzeichnet, daß der Antikörper TURP-27 mit den Kennzeichnungs-Charakteristiken von ATCC Nr. 40 292 ist.

**12.** Verfahren nach Anspruch 11, wobei das Heilmittel mit monoklonalem Antikörper einen an ein Arzneimittel gekoppelten Antikörper, einen an ein Toxin gekoppelten Antikörper, einen an ein Isotop gekoppelten Antikörper, einen bifunktionellen Heteroantikörper oder einen nativen Antikörper aufweist.

## Fig. 1A

## Fig. 1B

26

Fig. 2A

Fig. 2B

Fig. 3A   Fig. 3B